# EUROPEAN PATENT APPLICATION

(11) **EP 1 661 457 A1**
(43) Date of publication of application: **31.05.2006**
(21) Application number: 04772138.6
(22) Date of filing: 25.08.2004
(51) Int. Cl.: A01K 67/02

(54) **METHOD OF CONSTRUCTING OFFSPRING ORIGINATING FROM FROZEN SPERM STEM CELL**

(30) Priority: 27.08.2003 JP 2003303168
(71) Applicant: Kyoto University, Kyoto-shi, Kyoto 606-8501 (JP)
(72) Inventor: SHINOHARA, Takashi, Kyoto-shi, Kyoto 6068351 (JP); SHINOHARA, Mito, Kyoto-shi, Kyoto 6068351 (JP)
(74) Representative: Helbing, Jörg
(86) International application number: PCT/JP2004/012177
(87) International publication number: WO 2005/020679

(57) **Abstract**

The present invention provides a method of producing offspring originating in a spermatogonial stem cell of a donor animal which comprises generating spermatozoa in the reproductive organ of a male recipient animal with the use of frozen spermatogonial stem cells originating in the donor animal to give a male individual for reproduction, and then producing animal individual originating in the spermatogonial stem cell of the donor using the said individual for reproduction.

## Description

### TECHNICAL FIELD

The present invention relates to reproductive technology, more specifically, to a method of producing offspring originating from frozen spermatogonial stem cells.

### BACKGROUND ART

Currently, a method that uses cryopreserved sperm has become usable in the treatment of reproductive dysfunction of vertebrates such as human, breeding of domestic animals, preservation of species and the like. Actually, sperm- or embryo-cryopreservation is used in the line preservation of laboratory animals including mouse and domestic animals such as cattle. Generally, intended offspring are obtained in the following manner. First, sperm is freezed and preserved in liquid nitrogen and, after thawing, subjected to artificial insemination or *in vitro* fertilization. The resulting ovum is then transplanted into oviduct of pseudopregnant surrogate parent and an animal having the transplanted ovum is housed so as to obtain the objective offspring. However, preservation method for sperm differs depending on the species, and a commonly usable method has not been established yet. For example, there are no effective freezing method for sperm of C57BL/6(B6) mouse that is used in experiments. In addition, the fact that nitrogen supply is necessary for sperm cryopreservation not only makes the long-term preservation expensive but also requires one to pay special attention, for example, at the time of packaging for transportation. Besides, a given quantity of sperm is needed to attain fertilization, and hence a large quantity of sperm must be collected and cryopreserved because cryopreserved sperm cannot be grown *in vitro.* However, a large quantity of sperm can hardly be collected even from domestic animals from which sperm can be obtained with relative ease. It is particularly difficult to preserve a large quantity of human sperm in advance for the treatment of a subject in danger of infertility caused by testicular deficiency due to chemotherapy or irradiation therapy.

Further, in the case of immature individuals wherein sperm is not generated or those wherein spermatogenesis is inhibited for some reasons, germline cannot be preserved. In an attempt to use freeze-dried sperm, mouse sperm thawed with water was revealed to have the fertilizing capacity. However, said method has not been established yet because the fertilizing capacity decreases as the preservation term becomes longer, for example.

Spermatogenesis of animals generally involves a series of differentiation and development wherein germline stem cells (e.g., spermatogonia) in testes of human or corresponding male reproductive organs proliferate with morphological changes. In the case of human et al., spermatogonial stem cells (spermatogonia) develop to spermatocytes then to spermatids through meiosis, which spermatids undergo morphological changes to give sperm. Hereinafter, the present invention will be described taking spermatogonial stem cell as an example of germline stem cell.

Spermatogonial stem cells are, similar to other stem cells, not only stable during freeze-thaw and able to grow in *vitro* (Japan Patent Application No. 2003 -110821), but also highly resistant to irradiation, temperature, etc. and easy to handle at the occasion of preservation, transportation or the like. Spermatogonial stem cells have advantages including that they can be obtained from individuals lacking spermatogenesis or having a small amount of sperm, and that they proliferate in *vitro* to give a sufficient quantity of sperm even when only a small amount of sperm is available. Accordingly, if a reproduction technology to produce offspring (individual) originating in spermatogonial stem cell is established, it must be highly useful for breeding domestic animals, preserving rare species, treating human male infertility, fertility restoration of a patient in danger of infertility due to irradiation or chemotherapy, and the like. As for the freezing of spermatogonial stem cells, it has been known that cryopreservation can be carried out in almost the same manner while maintaining the spermatogenic activity across the species including hamster, rat, monkey, human, cattle, pig, and the like.

The transplantation technique for spermatogonial stem cells has been reported by Brinster et al. (Brinster, R. L. and Zimmermann, J. W. Spermatogenesis following male germ-cell transplantation. Proc. Natl. Acad. Sci. USA (1994) 91, 11298-11302), which technique comprises preparing cell suspension of spermatogonial stem cells and introducing into the seminiferous tubules of the testis. The document however is silent about the spermatogenesis from the frozen spermatogonial stem cells. Brinster et al. then confirmed *in vivo* that frozen spermatogonial stem cells have a spermatogenic activity (Avarbock, M. R., Brinster, C. J. and Brinster, R. L. Reconstitution of spermatogenesis from frozen spermatogonial stem cells. Nat. Med. (1996) 2, 693-696). However, it has not been reported that offspring was produced from a frozen spermatogonial stem cell. It was reported that microinsemination with spermatozoa generated by transplantation of cryopreserved testicular pieces into testis gave offspring originating in said spermatozoa (Shinohara, T et al., Birth of offspring following transplantation of cryopreserved immature testicular pieces and in-vitro microinsemination. Hum. Reprod. (2002) 17, 3039-3045); however, the testicular pieces contained Sertoli cells and the like. In addition, the microinsemination technique is expensive and requires specialized technique, and hence is not commonly applicable. Accoridingly, development of a method for producing offspring originating in spermatogonial stem cells economically in a reproducible fashion has strongly been demanded.

### DISCLOSURE OF THE INVENTION

One of the purposes of the present invention is to establish a method of producing offspring originating in spermatogonial stem cells (male germline stem cells) stably.

The present inventors have succeeded in producing offspring originating in spermatogonial stem cells of a donor animal by generating spermatozoa in the testis of a male recipient using the cryopreserved spermatogonial stem cells of the donor animal to obtain an individual for reproduction, and producing offspring with the use of said individual for reproduction, and established the present invention.

Thus, the present invention provides a method of producing offspring originating in a spermatogonial stem cell of a donor animal which comprises generating spermatozoa in the reproductive organ of a male recipient animal with the use of frozen spermatogonial stem cells derived from the donor animal to give a male individual for reproduction, and preparing an animal individual of the donor spermatogonial stem cell origin using the resulting male individual.

As one embodiment, the present invention provides a method of producing offspring originating in a spermatogonial stem cell of a donor animal which comprises transplanting frozen spermatogonial stem cells into the reproductive organ of a male recipient animal, generating spermatozoa in the recipient testis to obtain a male individual for reproduction, preparing a fertilized ovum with the use of a spermatozoon derived from the male individual, and allowing the resulting fertilized ovum to develop into an animal individual.

For the purposes of the present invention as disclosed in the description and claims, the donor and recipient male animals can be either the vertebrates or invertebrates.

The term "vertebrate" refers to mammals, birds, fishes, amphibians and reptiles. Preferred vertebrate includes a mammal selected from the group consisting of human, non-human primate, dog, cat, goat, pig, mouse, rat, sand rat, hamster, rabbit, pachyderm, horse, sheep, pig, cattle and marine mammal; and a bird selected from the group consisting of domestic duck, goose, turkey, chicken, ostrich, emu, guinea fowl, pigeon and quail, but is not limited thereto.

The term "invertebrate" refers to echinus, lobster, abalone and crustacean, but is not limited thereto.

For the present invention, it is preferred that the animal individual is vertebrate and the reproductive organ is testis.

The term "cryopreserved spermatogonial (male germline) stem cell" refers to a frozen preparation comprising spermatogonial stem cells having spermatogenic activity, and generally includes a thawed preparation. It would be clear from the context that which form, i.e., frozen or thawed form, the term refers to. In the present specification, the thawed "frozen spermatogonial stem cell" may be specifically referred to as "freeze-thawed spermatogonial stem cell".

Transplantation of frozen spermatogonial stem cells into a male recipient animal can be performed by providing a suspension of frozen spermatogonial stem cells to the seminiferous tubule or the rete testis.

The preparation of fertilized ovum using spermatozoa generated in a recipient male animal can be carried out through natural mating of the recipient male animal with a female animal; artificial reproduction wherein spermatozoa are injected into the genital canal of female individual; or in *vitro* fertilization-embryo transfer wherein spermatozoon of the recipient male animal and an ovum of a female animal are fertilized extracorporeally, and the resulting fertile ovum is grown for a given period of time ex vivo and implanted into the uterus. In case of in *vitro* fertilization, microinsemination can be employed.

In view of the purposes of the present invention, the male donor and recipient animals can be mature or immature animals. However, it is preferred that the male recipient is immature to obtain offspring through natural mating.

The method of the present invention can be effected using invertebrate animals as donor and recipient.

Because offspring can be obtained using a frozen spermatogonial stem cell according to the present invention, it is possible to collect spermatogonial stem cells at an appropriate time, cryopreserved, optionally grown *in vitro* and further cryopreserved, and then subjected to the production of offspring as needed. Since the spermatogonial stem cells exist even in an individual lacking spermatogenesis, spermatogonial stem cells can be isolated from an immature individual and preserved, and then transplanted into the same or an allergenic individual to generate sperm, whereby it became possible to produce offspring originating in the spermatogonial stem cell. In the case of an individual in danger of infertility due to degradation or impair of spermatogenic capacity as the result of chemotherapy or exposure to endocrine disrupter, it is also possible to obtain offspring originating in spermatogonial stem cells that have been collected from the individual and stored.

As described above, the present invention makes it possible to preserve lines of rare species, laboratory animals, domestic animals and the like, and restore infertility human patients suspected to become infertile due to chemotherapy, irradiation, or the like. In addition, as offspring originating in frozen spermatogonial stem cell can be obtained through natural mating, the production of offspring is achievable economically without any technology or equipments required for the microinsemination.

### BRIEF DESCRIPTION OF FRAWINGS

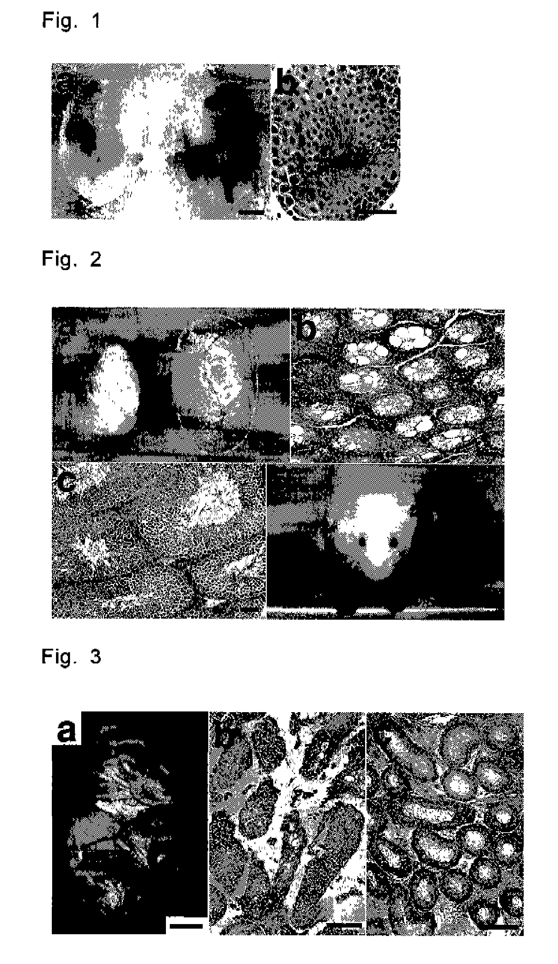

Figure 1 shows the comparison of the stem cell activities of fresh and freeze-thawed ROSA26 testis cells after transplantation into infertile W recipient mice. (a) Macroscopic appearance of recipient testes after transplantation of fresh (3x10⁵ cells injected; left) or frozen-thawed (3x10⁴ cells injected; right) ROSA 26 testis cells. (b) Photomicrograph of a histological section of a recipient testis 2 months after transplantation with freeze-thawed stem cells.

Figure 2 shows the fertility restoration of infertile W mice after transplantation of freeze-thawed testis cells. (a) A macroscopic comparison of untransplanted mouse (left) and transplanted recipient (right) testes 241 days after transplantation with frozen stem cells (mouse #1156). (b) Photomicrograph of a histological section of the untransplanted W recipient testis. (c) Photomicrograph of a histological section of the transplanted recipient testis. (d) Offspring from an infertile W recipient male (white, # 1156) that was transplanted with freeze-thawed, cryptorchid testis cells from a Green mouse.

Figure 3 is a photograph showing the donor testis cell colonization in the busulfan-treated, adult recipient testis. (a) Macroscopic appearance of a recipient testis that received immature green mouse testis cells. (b) Photomicrograph of a histological section of the testis shown in (a). (c) Tubules from epididymis of the testis shown in (a).

### BEST MODE FOR CARRYING OUT THE INVENTION

The present invention will be hereinafter described taking mice as an example; however, one of ordinary skilled in the art would easily understand that the present invention can be put into practice using mammals other than mouse and further vertebrates and invertebrates with the use of the method described herein or that known in the art.

The method for preparing frozen spermatogonial stem cells and thawing the same is substantially the same as that used for other stem cells (Avarbock, M. R. et al., Nat. Med. (1996) 2, 693-696). Specifically, the preparation can be performed by the method described in a text (Jikken-igaku bessatsu (Experimental Medicine, separate volume) "Stem cell clone, research protocol" Yodosha).

When a sample is collected from an adult donor of which testis contains a small amount of germline stem cells (i.e., undifferentiated cells), it is preferred to be concentrated according to a known method (Jikken-igaku bessatsu, ibid.) before freezing. When a sample is collected from an immature donor of which testis contains a large amount of male germline stem cells, it can be used without concentration. For example, in the case of mouse, about 1-week-old immature individual is preferably used for preparation. Further, spermatogonial stem cells can be proliferated in *vitro* before freezing (Japan Patent Application No. 2003― 110821).

In the method of the present invention, frozen spermatogonial stem cells prepared by any method can be used. For example, frozen spermatogonial stem cells can be prepared by isolating the whole or a part of testis from a donor animal, removing the tunica albuginea in a solvent (PBS: Phosphate-Buffered Saline), dissociating the cells with collagenase, trypsin and DNase, suspending the testis cells separated into single cells in a cell cryopreservation solution (Cellbanker; DIA-IATRON, Tokyo) containing dimethyl sulfoxide and fetal bovine serum albumin, transferring aliquots of approximately 1 ml of the cell suspension containing 5x10⁶-10⁷ cells to 1.5 ml cryotubes, and subjecting to freezing at -80 °C for 1 day. The resulting frozen spermatogonial stem cells can be preserved in liquid nitrogen at -196°C.

Frozen spermatogonial stem cells can be grown *in vitro* and cryopreserved again during the preservation period, and be preserved almost forever. At the time of use, frozen cells are thawed and suspended in a solvent to give a cell suspension in a conventional manner. Any methods can be used for thawing without limitation. For example, thawing is performed in a water-bath at 37 °C using Dulbecco's modified Eagle's medium (DMEM) containing 10% fetal calf serum (DMEM/FCS). Specifically, frozen cells are thawed by adding 10 ml of DMEM/FCS into cryotubes floated in a water-bath. After washing by centrifugation, cells are suspended in DMEM/FCS and kept on ice until transplantation.

The transplantation of spermatogonial stem cells with the use of a stem cell suspension into recipient testis can be performed by any methods known in the art. For example, transplantation can be performed by direct injection into seminiferous tubules through microinjection or by injection into efferent ducts through microinjection thereby allowing to reach rete testis. The latter is preferred. The transplanted spermatogonial stem cells adhere to the tube wall of the recipient seminiferous tubules, and then differentiate and develop into spermatocytes, spermatids and spermatozoa, and finally mature following transfer to the epididymis.

A fertile ovum inseminated with a spermatozoon generated in the recipient testis can be obtained through natural mating with a male animal, artificial insemination, or in *vitro* fertilization-embryo transfer in a conventional manner. The in *vitro* fertilization-embryo transfer can be carried out by microinsemination. Those methods are known in the art. When a female animal having a fertile ovum (ova) is kept under appropriate conditions for a certain period of time, offspring of the male donor origin can be obtained.

In the above method, the donor and recipient male animals may be the same or different individual.

The present invention is further illustrated by the following examples, but should not be limited by them in any respect.

### EXAMPLES

In the Examples below, the colonization capacity of frozen and fresh spermatogonial stem cells prepared from donor mouse testis were compared (quantification, first experiment), and then offspring originating in frozen spermatogonial stem cells were produced (second experiment).

Donor and recipient mice and methods used for the preparation of donor cells (freeze-thawed spermatogonial stem cells), transplantation of donor cells, histological evaluation of recipient testis and microinsemination employed in the Examples are described below.

### (1) Mouse

### 1) Donor mouse (first experiment)

ROSA: B6-TgR(ROSA26)26Sor transgenic mouse purchased from Jackson Laboratory, USA

### Characteristics:

ROSA26 mouse expresses the *E.coli LacZ* transgene in all of the spermatogenic cells in the seminiferous tubules. The transplanted donor testis cells express β -galactosidase, and hence are stained blue (LacZ staining) in the presence of the substrate 5-bromo-4-chloro-3-indolyl β -D-galactosidase (X-gal). Since the donor mouse expresses the *LacZ* transgene in all of the spermatogenic cells, the spermatogenesis originated from the donor stem cells can be detected by LacZ staining (blue) with the substrate X-gal.

### 2) Donor mouse (second experiment)

GFP (Green): C57BL/6 Tg14(act-EGFP)OsbY01 transgenic mouse line provided by Dr M.Okabe (Osaka University) (Okabe et al., 1997, FEBS Lett. 407,313-319)

### Characteristics:

The spermatogonia and spermatocytes of GFP mice express the gene for the enhanced green fluorescent protein (EGFP), the amount of which gradually decreases after meiosis. Accordingly, the testis cells can be detected on the basis of fluorescence intensity. Colonies positive for the EGFP transgene originating in the donor testis cells were observed with a fluorescent stereomicroscope (MZ FLIII, Leica) in the examples below. In the experiments, male pups (pup, 6-day-old) or adult mice (adult, 6-8 weeks of age) positive for the transgenes were used. Donor testis cells were collected from the testes of 6-day-old Green mouse pups or from the cryptorchid testes of adult Green mice, 2-3 months after the operation. It has been shown that these testes are enriched for stem cells, due to the absence of differentiated germ cells, donor testis cells obtained from these mice should improve colonization efficiency and facilitate fertility restoration. Cryptorchid testes were produced as previously described (Shinohara et al., 2000, Dev. Biol. 220, 401-411).

### 3) Recipient mouse (first and second experiments)

B6 mouse (busulfan-treated or -untreated): C57BL/6 (B6) mouse (6-12 weeks old) purchased from the Shizuoka Laboratory Animal Center (SLC, Japan)

### Characteristics:

B6 mice (busulfan-treated) were treated with busulfan (44 mg/kg bodyweight) at 6 weeks of age to destroy the endogenous germ cells responsive to spermatogenesis, and used in transplantation 1 month after busulfan injection. Busulfan treatment allows donor cell colonization by destroying the endogenous spermatogonial stem cells. Thus, it mimics the side effects of chemotherapy that occurs in oncology patients.

### W mouse: WBB6F1-W/Wv mouse (5-10 days old or 6-12 weeks old, Japan SLC)

### Characteristics:

W mice lack endogenous spermatogenesis due to mutations in the c-kit tyrosine kinase gene which is normally expressed on germ cells.

Both the busulfan-treated B6 mice and the congenitally infertile W mice have been shown to be capable of generating spermatogenesis from transplanted fresh stem cells (Ogawa, T. et al.(2000) Nat. Med. 6, 29-34; Shinohara, T. et al., Dev. Biol. 220, 401-411).

### (2) Donor cells (spermatogonial stem cells derived from donor); General procedures

1) The process generally comprises isolating testis from a donor mouse, and removing the tunica albuginea in PBS, and incubating in Hanks' balanced salt solution containing 1 mg/ml collagenase (Type I) and 7mg/ml DNase at 37°C for 15 minutes while shaking as appropriate to digest the seminiferous tubules. After removing the separated stromal cells by washing (x2) with PBS, the seminiferous tubules were incubated in PBS containing 0.25% trypsin at 37°C for 15 minutes while shaking as appropriate to further digest the seminiferous tubules. Trypsin was then inactivated by adding PBS, followed by pipetting to yield a cell suspension. The suspension was filtered through a nylon mesh (20-30 µm) to remove the undigested cell masses, and centrifuged (600xg) for 5 minutes to collect the cells.

When a fresh cell preparation was used for transplantation, a cell suspension was prepared by suspending the resulting cells in DMEM/FCS.

Cryopreservation of cells were performed by suspending the single testis cell obtained above in Cellbanker (DIA-IATRON, Tokyo) containing dimethyl sulfoxide and fetal bovine serum albumin, transfering aliquots of 1 ml of the cell suspension (cell density: 10⁷/ml) to 1.5 ml cryotubes, and freezing at -80 °C for 1 day. The frozen spermatogonial stem cells were preserved in liquid nitrogen at -196°C until use.

### 2) Thawing of frozen donor cells

Thawing was carried out substantially in accordance with the teaching of the supplier of Cellbanker. That is, 10 ml of Dulbecco's modified Eagle's medium (DMEM) containing 10% FCS (DMEM/FCS) was added dropwise into cryotubes floated on a water-bath at 37°C. After washing by centrifugation (600g x5 minutes), the cells were resuspended in DMEM / FCS and kept on ice until transplantation.

### (3) Transplantation of donor cells

Transplantation was performed by introducing a donor testis cell suspension in DMEM/FCS into the seminiferous tubules or the efferent ducts of a recipient mouse by microinjection in a conventional manner (see, Jikken-igaku bessatsu (Experimental Medicine, separate volume) "Stem cell clone, research protocol" Yodosha). The adult (mature) mice were anaesthetized with Avertin injection (640 mg/kg) and the cells were transplanted into the testes of both sides. The pup (immature) mice were placed on ice to cause hypothermia-induced anesthesia and the cells were transplanted into the testis of only one side. This is to avoid decrease of postoperative survival rate by the long-term hypothermia. The experimental design is summarized in Table 1 below. In the experiments with B6 mice, approximately 10 µl of the donor testis cell suspension was introduced into the seminiferous tubules or the efferent ducts by microinjection, whereas only 3 µl of the suspension was injected into the adult W testis as the testis is small. When immature W recipients were used as recipients, 2 µl of the donor testis cell suspension was injected. In each testis, 75-85% of the tubules were filled with the donor cells.

In the transplantation of the first experiments, frozen donor cells were suspended in DMEM/FCS at a concentration of 7.5x10⁶ to 3.0x10⁷ cells/ml, whereas the concentration of fresh cells was 10⁸ cells/ml, because the recovery of frozen-thawed cells varied between experiments.

In the second experiments, the frozen donor cells were suspended in DMEM/FCS at a concentration of either 10⁸ cells/ml (B6 or mature W mice) or 3x10⁷ cells/ml.

### (4) Histological analysis

The histological analysis of recipient mouse testes was performed by macroscopic or microscopic analysis. The microscopic analysis was carried out in the following manner.

Testis tissue was fixed in 10% neutral buffered formalin, embedded in paraffin wax and then cut into sections at 12 µm intervals. All sections were stained with haematoxylin and eosin. Four histological sections were taken from each testis. Each slide was viewed at a magnification of x400 for the analysis. In the second experiment, the numbers of tubule cross-sections with evidence of spermatogenesis (defined as the presence of multiple layers of germ cells in the entire circumference of the seminiferous tubule) or lacking evidence of spermatogenesis were recorded for three sections from each testis to assess the level of spermatogenesis in the recipient testis. At least 500 seminiferous tubules were counted, and statistical analyses were performed using Student's t-test.

### (5) Microinsemination

Microinsemination was performed by injecting donor testis cells microscopically into oocytes collected from superovulated female mice (C57BL/6xDBA/2 F1) as described (Kimura, Y. et al. (1995) Development 121, 2397-2405). Embryos that reached the 2-cell stage after 24 hours in culture were transferred to the oviducts of pseudopregnant ICR females.

The experimental design for the first and the second experiments is summarized in Table I.

**Table I: Experimental Design for the First and the Second Experiments.**

| Ex.^{a} | Donor^{b} | Recipient^{c} | Type cells | Donor cell of concentration (x10⁶cell/ml) (µl) | Volume No. injected | of recipient |
|---|---|---|---|---|---|---|
| 1^{st} Ex. | Rosa26, adult, untreated | W, adult | Frozen | 7.5-30 | 3 | 6 |
| | | B6, adult, busulfan busulfan | Frozen | 7.5-30 | 10 | 4 |
| | | W, adult | Fresh | 100 | 3 | 6 |
| | | B6, adult, busulfan | Fresh | 100 | 10 | 4 |
| 2^{nd} Ex. | GFP, adult, | B6, adult, busulfan | Frozen | 100 | 10 | 12 |
| | cryptorchid | W, adult | Frozen | 100 | 3 | 9 |
| | | W, pup | Frozen | 30 | 2 | 7 |
| | GFP, pup | W, pup | Frozen | 30 | 2 | 1 |

a: First experiments were designed to evaluate effect of freezing on spermatogonial stem cells; second experiments were designed to derive offspring from freeze-thawed spermatogonial stem cells.
b: ROSA A26 adult: 6-8 weeks old; GFP adult: 14-20 weeks old; GFP pup: 6 days old.
c: W adult: 6-12 weeks old; B6 adult: 10-12 weeks old; W pup: 5-10 days old.

### Example 1: Quantification of Spermatogonial Stem Cells (First Experiment)

The quantity of spermatogonial stem cells in the testis cells collected from donor mouse was determined.
(1) Adult ROSA26 was used as the donor. The freeze-thaw of donor testis cells and the preparation of cell suspension thereof were performed according to the method described above in "(2) Donor cells (spermatogonial stem cells originated in donor); General procedures". As above-mentioned, spermatozoa originating in spermatogonial stem cells of ROSA26 develop blue color upon LacZ staining with X-gal, which makes it possible to quantify spermatogonial stem cells in donor testis cell preparation.
(2) Transplantation of donor testis cells

### Preparation of cell suspension

A cell suspension of freeze-thawed donor testis cells in DMEMFCS which contains thawed donor testis cells having been cryopreserved for 5 months in liquid nitrogen, and a cell suspension of fresh donor testis cells in DMEM/FCS were used. The viability of cells in the freeze-thawed or fresh cell preparation was examined by trypan blue exclusion.

The viability index of freeze-thawed testis cells was, as indicated by trypan blue exclusion, significantly reduced compared with that of fresh cells not underwent freeze-thawing treatment (viability index of freeze-thawed cells: 67.4 ± 5.9%, mean ± SEM, n = 8; fresh cells: 93.6 ± 1.7%; , mean ± SEM, n = 7; P < 0.01). The average recovery for the originally frozen cell population was 37.6 ± 5.1% (n = 8).

In the frozen-thaw preparations, only live donor testis cells were counted for transplantation.

### Recipients

Congenitally infertile W mice (5-10 days old or 6-12 weeks old) and busulfan-treated B6 mice (6-12 weeks old) were used.

To examine spermatogenic activity of frozen-thawed stem cells, the same number of cells were transplanted to respective recipients. The donor testis cells were used as a suspension in DMEM/FCS.

B6 mice were transplanted with 3 *µ*l suspension of freeze-thawed donor testis cells (7.5x10⁶ to 3.0x10⁷ cells/ml) or 3 *µ*l suspension of fresh donor testis cell (1.0x10⁷ cells/ml).

As described in (4) above, 10 *µ*l each of stem cell suspension was introduced into the testis of each recipient under anesthesia by Avertin injection (640 mg/kg).

Mice were kept under the normal conditions for rearing for 2 months and underwent laparotomy. The testis of each mouse was isolated and stained with X-gal. Each blue-stained region of the seminiferous tubules (colonies) represented spermatogenesis from transplanted single spermatogonial stem cell (Nagano, M.et al. (1999) Biol. Reprod. 60, 1429-1436). That is, the other testis cells are irrelevant to spermatogenesis and the endogenous recipient germ cells do not stain positive with X-gal. Therefore, the number of blue colonies represents the number of stem cells in the transplanted cell population.

The results are shown in Table II and Figure 1. Figure 1 shows the comparison of stem cell (colonization) activities of fresh and freeze-thawed ROSA 26 testis cells after transplantation into infertile W recipient mice. (a) Macroscopic appearance of W recipient testes after transplantation of fresh (3x10⁵ cells injected; left) or frozen-thawed (3x10⁴ cells injected; right) ROSA 26 testis cells. The blue coloration represents donor-derived spermatogenesis, which can be seen as gray/black spots in the black-and-white picture. The level of donor cell-derived spermatogenesis from freeze-thawed donor testis cells is higher than that from fresh donor testis cells as can be seen from Fig.1 which shows that the number of spermatogenesis in testis is one for the fresh cells (Fig. 1a, left) while that is 4 for the freeze-thawed cells (Fig. 1a, right). It is clear that the freeze-thawed donor cells exhibit increased level of donor cell-derived spermatogenesis despite lower concentration of injected cells. (b) Photomicrograph of a histological section of a recipient (W) testis 2 months after transplantation with freeze-thawed testis cells. The picture shows the normal appearance and organization of the germ cells. Haematoxylin and eosin staining was conducted. Scale bars: (a) = 1 mm; (b) = 25 µm.

**Table II**

| | W recipients | | Busulfan-treated recipients | |
|---|---|---|---|---|
| Type of cells transplanted | No. of tubule s injected | No. or colonies No. or colonies | No. of tubules injected | No. of colonies |
| Freeze/thawed | 11 | 62.1±21.7 | 8 | 45.9±16.2 |
| Fresh | 11 | 5.3±2.2 | 8 | 9.0±3.2 |

Values are mean ± SEM. The results are based on data from two experiments for eache set. Approximately 3 µl was injected into W mice testes, whereas 10 µl was injected into busulfan-treated B6 mice testes.

Table II shows that, in the case of W recipient mice, the extent of colony generation from freeze-thawed (frozen) donor testis cells was 11.7-times that from fresh donor testis cells (62.1 versus 5.3 colonies per 3 x 105 donor cells; P < 0.05). Likewise, in the case of busulfan-treated B6 recipient mice, the number of colonies from freeze-thawed donor testis cells was 5.1-times higher than that from fresh donor testis cells (P < 0.01). The results shown in Figure 1 and Table II, when taken together, demonstrate that freeze-thawed testis cells have higher spermatogenic activities than fresh donor cells. These results also indicate that the method of the present invention makes it possible to perform fertilization through natural mating as a large quantity of spermatozoa can be produced stably, and is suited to a practical use.

### Example 2: Fertility Restoration of Recipients Transplanted with Frozen Spermatogonial Stem Cells and Production of Offspring Originating in the Donor Stem Cells (Second Experiment)

(1) The above-mentioned transgenic mice GFP (Green) were used as a donor. The freeze-thawing of donor testis cells and preparation of cell suspension of donor testis cells were performed according to the method described above in "(2) Donor cells (spermatogonial stem cells originated in donor); General procedures" Spermatogonial stem cells were collected from the testis of 6-day-old mouse (pup) or from the cryptorchid testes of adult Green mice, 2-3 months after the operation, and frozen. The spermatogonia and spermatocytes of these transgenic mice express the gene for the enhanced green fluorescent protein (EGFP), the amount of which gradually decreases after meiosis.
(2) Transplantation of donor cells

### Donor cells for transplantation

A cell suspension used was prepared according to the method described above by preserving frozen donor testis cells in liquid nitrogen for 2-3 weeks, thawing and suspending in DMEM/FCS.

### Transplantation

Transplantation was performed according to the method described above in "(3) Transplantation of donor cells".

As designed in Table I, a suspension of donor testis cells from the cryptorchid testes of adult GFP mice was introduced into B6 mice (adult, busulfan-treated) and W mice (adult or pup) by microinjection. A suspension of donor testis cells from pup GFP mice was introduced into W mice (pup) by microinjection. Following the transplantation of donor testis cells, the recipient mice were kept under normal conditions for the animal. As a control, untransplanted animals were kept under the same conditions.

### Production of offspring by natural mating

The recipient mice which had been transplanted with donor testis cells and kept under the conditions above were housed with B6 wild-type females under the normal conditions for these animals to cause natural mating, and the generation of offspring was examined. The adult and pup mice were subjected to natural mating after 2 weeks and 6 weeks later, respectively.

### Histological analysis of recipient testes

The recipient mice were kept under the normal conditions and subjected to laparotomy 213 - 246 days after the transplantation of donor testis cells. The testes were isolated from respective mice and subjected to the evaluation of donor cell colonization efficiency. Histological sections of testis were obtained in a similar manner to Example 1, and each slide was viewed at a magnification of x400 with an upright microscope for the analysis. To assess the level of spermatogenesis in the host testis, the numbers of tubule cross-sections with evidence of spermatogenesis (defined as the presence of multiple layers of germ cells in the entire circumference of the seminiferous tubule) or lacking evidence of spermatogenesis were recorded for three sections from each testis. At least 500 seminiferous tubules were counted. Statistical analyses were performed using Student's t-test.

The testis of fertile recipients which sired offspring, infertile recipients which failed to sire offspring and control animals which were simply housed without transplantation was weighted.

The results are shown in Tables III, IV and Figure 2. Figure 2 shows the results of experiments for fertility restoration of infertile W mice by the transplantation of freeze-thawed testis cells. (a) A microphotograph of untransplanted (left) and transplanted (right) recipient testes 241 days after transplantation with frozen spermatogonial stem cells (mouse #1156). The figure shows that the transplanted recipient testis is considerably larger compared to the untransplanted testis. (b) A microphotograph of histological section of the untransplanted W recipient testis. (c) A microphotograph of histological section of the transplanted recipient testis. In Fig. 2 (b), tubules are blank (white) showing that no spermatozoa exist. On the contrary, Fig. 2(c) shows that many spermatozoa are generated as indicated by the black spots in the entire circumference of tubules. (d) Offspring from a B6 female (black) mated with an infertile W recipient male (white, 1156) undergone transplantation with freeze-thawed, cryptorchid testis cells from a Green mouse. Haematoxylin and eosin staining was performed. Scale bars: (a) = 1 mm; (b, c) = 50 µm.

Table III shows the combined results from two to three separate experiments for each type of transplant. Values are mean ± SEM.

Table IV shows the detailed data of five recipients that restored fertility.

**Table III: Spermatogenesis after transplantation of freeze-thawed testis cells**

| Recipient | | | % Tubules with spermatogenesis^{a} | No. of epididymides with spermatozoa (%) | No. of fertile recipients |
|---|---|---|---|---|---|
| Type | Age | No. | | | |
| W | pup | 8 | 54.5 ± 8.9 | 7 (87.5) | 4 |
| W | adult | 9 | 38.4 ± 6.3 | 5 (55.6) | 1 |
| B6 busulfan | adult | 12 | N. D^{b} | N. D^{b} | 0 |

| | | | | | |
|---|---|---|---|---|---|
| a: Percentage of tubule cross-sections in the recipient testis with spermatogenesis. Seminiferous tubule cross-sections with multiple layers of germ cells were considered to be positive for spermatogenesis. b: N.D. = not determined (due to the presence of endogenous spermatogenesis). | | | | | |

**Table IV: Progeny from W recipient mice microinjected with freeze-thawed stem cells**

| Fertile recipient | | Donor cell type^{a} | Recipient | Days to analysis | Testis weight^{b} | %Tubule cross- sections with (mg) spermatogenesis^{c} | Days to first progeny^{d} |
|---|---|---|---|---|---|---|---|
| 1150 | R | adult | pup | 241 | 44.7 | 62.7 | 190 |
| | L | | | | 11.3 | N.D. | |
| 1156 | R | adult | pup | 241 | 58.5 | 87.6 | 81 |
| | L | | | | 12.0 | N.D. | |
| 1860 | R | adult | pup | 228 | 23.9 | 87.7 | 72 |
| | L | | | | 6.5 | N.D. | |
| 1868 | R | pup | pup | 240 | 35.8 | 67.0 | 136 |
| | L | | | | 11.5 | N.D | |
| 2711 | R | adult | adult | 221 | 33.7 | 65.9 | 221 |
| | L | | | | 32.2 | 59.2 | |

| | | | | | | | |
|---|---|---|---|---|---|---|---|
| a: Donor cell type: Adult, cryptorchid. b: Only right testes were injected with cells in the pup recipients, whereas both testes were injected in the adult recipients. c: The rate (%) of recipient testis cross-sections with spermatogenesis. Seminiferous tubule cross-sections with multiple layers of germ cells were considered to be positive for spermatogenesis. d: Days from transplantation to birth of first progeny sired by the female mice. N.D. = not determined, because donor cells were not transplanted. | | | | | | | |

Table III shows that four of the eight W (pup) recipient mice having been transplanted with the freeze-thawed donor testis cells restored fertility and sired offspring within 72-190 days of transplantation. As shown in Table IV, three of the fertile males received adult cryptorchid donor testis cells, and one of the fertile males received pup donor testis cells. The donor testis cell origin of the offspring was confirmed by green fluorescence under ultraviolet (UV) light.

The mean weight of the fertile recipient testes was significantly higher (40.7 ± 7.3 mg; n = 4) than that of the infertile recipient testes (22.6 ± 2.2 mg; n = 4; P < 0.05) or that of the untransplanted control testes (10.4 ± 0.8 mg; n = 7; P < 0.01) (Figure 2a). Histological analyses also revealed more extensive donor germ cell colonization of the fertile recipient testes (77.4 ± 4.8%; n = 4) than of infertile recipient testes (27.0 ± 2.3%; n = 4; P < 0.001) (Figure 2b and c).

Spermatogenesis in the recipient testes arose exclusively from donor stem cells, as the stem cells in the W recipient could not undergo spermatogenesis. However, the restoration of spermatogenesis occurred in all eight immature W recipient testes, and spermatozoa were observed in 87.5% (7/8) of epididymis sections, which suggests potential fertility. The four recipients that produced progeny remained fertile up to the time of analysis; that is, at least 228 days after transplantation (Figure 2d; Table 3), which indicates that the transplanted stem cells underwent continuous division and normal differentiation.

Taken together, these results demonstrate that the transplantation of frozen stem cells from the testes of pups or cryptorchid adults restored normal fertility to congenitally infertile W recipients.

By contrast, the restoration of fertility was not so efficient in adult recipients. Although one of nine adult W recipients achieved fertility through natural mating, the offspring was first obtained 221 days after transplantation. The recovery of spermatogenesis was observed in all the cases, but the percentage of the epididymides containing spermatozoa was smaller as compared with the recipient pups (55.6 versus 87.5%, Table III). In addition, none of the busulfan-treated recipients became fertile after 7 months.

Figure 3 shows donor testis cell colonization in the busulfan-treated, adult recipient testis. (a) A macroscopic appearance of a recipient testis that received Green pup testis cells. Green seminiferous tubules under UV light indicate donor testis cell colonization, which can be seen as white/gray image in the black-and-white picture. (b) A microphotograph of histological section of the testis shown in (a). Spermatogenesis is observed in several tubules. However, as shown in (c), tubules from epididymis of the mouse with testis shown in (a) contains no spermatozoa. Haematoxylin and eosin staining were used. Scale bars: (a) = 1 mm; (b, c) = 200 µm.

Donor-derived spermatogenesis occurred in the testes of both types of recipients (W, B6); however, the level of spermatogenesis was significantly lower than that in the pup recipients. As can be seen from the picture of epididymis, these recipients have smaller number of spermatozoa and therefore could not make female mice pregnant through natural mating. This is also apparent from the histological analysis of the busulfan-treated recipient testes examined 12 months after transplantation (Figure 3b and c).

### Microinsemination

In order to overcome the infertility of the busulfan-treated recipient mice which are infertile in natural mating, an in-vitro microinsemination technique was employed which is commonly used to derive offspring from infertile humans (Palermo, G. *et al*., (1992) Lancet 340, 17-18; Kimura, Y. et al., (1995) Development 121, 2397-2405). One of the busulfan-treated recipient mice was sacrificed 180 days after transplantation with frozen-thawed donor testis cells (pup donor-derived), and colonies positive for the EGFP transgene originating in the donor testis cells were observed with a fluorescent stereomicroscope (MZ FLIII, Leica). Live spermatogenic cells were recovered by repeatedly pipetting colonized tubules. The germ cells thus recovered were frozen according the previously reported method and stored until microinsemination. After 3-day-storage, the mature spermatozoa or elongated spermatids were injected into female mice (C57BL/6xDBA/2 F1 (B6D2F1)) oocytes (cytoplasm). The oocytes were collected from superovulated females. About 80% of oocytes developed into 2-cell forms within 24 h irrespective of the male germ cells used. All 101 diploid zygotes constructed with 80 spermatozoa and 21 elongated spermatids were transferred into the oviducts, 68 (67%) of which were implanted into the uterus and 31 pups in total were born (31% efficiency). Donor origin was confirmed by fluorescence under UV light. The offspring were proven to be fertile.

As described above, it was demonstrated that offspring originating in spermatogonial stem cells can be obtained through natural mating by allowing mature (adult) or immature (pup) recipients to generate spermatozoa using the spermatogonial stem cells derived from immature or mature individuals. The results also demonstrate that the rate of success for generating offspring through natural mating tends to become higher in case of immature recipients compared to mature recipients.

### INDUSTRIAL APPLICABILITY

According to the present invention, it became possible to use frozen spermatogonial stem cells in the reproductive technology for vertebrates in place of frozen sperm, which dissolves various problems associated with the method using frozen sperm, for example, complicated procedures required for freezing and preservation of sperm, decrease of reproductive potential of thawed sperm, difficulty in collecting abundant sperm, and the like. Thus, the present invention opens the way for establishing the reproductive technology with improved certainty and stability.

The method of the present invention is useful in not only preservation of lines of domestic or laboratory animals, but also protection of rare animals and treatment of human suffering from congenital infertility or acquired infertility due to treatment of malignant tumors or the like.

## Claims

1. A method of producing offspring originating in a spermatogonial stem cell, which comprises generating spermatozoa in the reproductive organ of a male recipient animal with the use of frozen spermatogonial stem cells derived from a donor animal to give a male individual for reproduction, and producing an animal individual originating in the donor spermatogonial stem cell using the said individual for reproduction.

2. The method of claim 1, wherein the frozen spermatogonial stem cells originating in the donor animal is transplanted into the reproductive organ of a male recipient animal, allowing to generate spermatozoa in the recipient testis to give a male individual for reproduction, producing a fertilized ovum using the spermatozoa derived from the said individual, and developing the resulting fertilized ovum into an animal individual.

3. The method of claim 2, wherein the fertilized ovum is obtained by natural mating of the male individual for reproduction with a female individual, or artificial insemination or in *vitro* fertilization-embryo transfer.

4. The method of claim 3, wherein the fertilized ovum is obtained by natural mating of the male individual for reproduction with a female animal.

5. The method of any one of claims 1 to 4, wherein the male recipient animal is immature animal.

6. The method of any one of claims 1 to 5, wherein the donor and recipient animals are vertebrates.

7. The method of claim 6, wherein the vertebrate is selected from mammals, birds, fishes, amphibians and reptiles.

8. The method of any one of claims 1 to 5, wherein the donor and recipient animals are invertebrates.

9. The method of any one of claims 1 to 8, wherein the frozen spermatogonial stem cells are introduced into the seminiferous tubule or the rete testis of the male recipient animal

10. The method of claim 9, wherein the frozen spermatogonial stem cells are introduced into the rete testis through the seminiferous tubule as a guide of the male recipient animal
